# EUROPÄISCHE PATENTSCHRIFT

(11) **EP 1 196 237 B1**
(45) Veröffentlichungstag und Bekanntmachung des Hinweises auf die Patenterteilung: **12.03.2003**
(21) Anmeldenummer: 00954471.9
(22) Anmeldetag: 11.07.2000
(51) Int. Cl.: B01J 2/26, C07C 29/17, C07C 29/42, C07C 29/76

(54) **VERFAHREN ZUR HERSTELLUNG EINES GRANULATS AUS EINER SCHMELZE**
METHOD FOR PRODUCING A GRANULATE FROM A MELT
PROCEDE POUR PRODUIRE UN GRANULAT A PARTIR D'UNE MATIERE FONDUE

(30) Priorität: 15.07.1999 DE 19933289
(43) Veröffentlichungstag der Anmeldung: 17.04.2002
(73) Patentinhaber: BASF AKTIENGESELLSCHAFT, 67056 Ludwigshafen (DE)
(72) Erfinder: BRUNNER, Melanie, D-68165 Mannheim (DE); LORENZ, Rudolf, Erich, D-67069 Ludwigshafen (DE); MALTRY, Bernhard, D-67283 Obrigheim (DE); HEILEK, Jörg, D-69245 Bammental (DE)
(74) Vertreter: Isenbruck, Günter, Dr.
(86) Internationale Anmeldenummer: EP0006576
(87) Internationale Veröffentlichungsnummer: WO01005495

(56) Entgegenhaltungen:
- EP-A- 0 884 097
- FR-A- 950 894
- US-A- 2 488 082
- US-A- 5 741 519
- DATABASE WPI Section Ch, Week 199402 Derwent Publications Ltd., London, GB; Class D21, AN 1994-013829 XP002156256 & KR 9 304 592 B (LUCKY CO LTD), 1. Juni 1993 (1993-06-01)

## Beschreibung

Die vorliegende Erfindung betrifft ein Verfahren zur Herstellung eines Granulats aus einer Schmelze sowie das Granulat selbst.

Verfahren zur Herstellung von Granulaten sind im Stand der Technik bereits beschrieben. Zahlreiche dieser Verfahren weisen jedoch Nachteile wie beispielsweise einen hohen apparativen Aurwand auf. Dieser apparative Aufwand ist vor allem dann besonders hoch. wenn es darum geht, Granulate mit beispielsweise pastillenförmiger Geometrie zu erzeugen.

Die DE-C 32 09 747 beispielsweise beschreibt ein Verfahren zur Herstellung eines Granulats aus einer Schmelze, in dem der Kristallgehalt der Schmelze von ganz entscheidender Bedeutung ist. Um diesen Kristallgehalt möglichst gut steuern zu können, wird ein Vorkristallisator eingesetzt, in dem die Schmelze mit Kristallkeimen angereichert werden muß.

Die DE-C 196 37 380 offenbart die Herstellung von Hydroxypivalinsäureneopentylglykolester-Granulaten. Auch bei diesem Verfahren ist der Gehalt der Schmelze an Kristallen von wesentlicher Bedeutung. Der Kristallgehalt wird in diesem Verfahren entweder dadurch erreicht, daß der Schmelze zusätzlich Feststoff zugegeben wird oder, wie in der DE-C 32 09 747, ein Vorkristallisator verwendet wird. In diesem Vorkristallisator muß ein 2-Phasengemisch mit einem bestimmten Gehalt an Kristallen hergestellt werden. In diesem Fall ist der apparative Aufwand ebenfalls sehr hoch, da zusätzlich zur Vorkristallisation Maßnahmen ergriffen werden müssen, die einen gewissen Homogenitätsgrad des 2-Phasengemisches sicherstellen sollen.

Eine Aufgabe der vorliegenden Erfindung war es daher, ein Verfahren bereitzustellen, das die Herstellung eines pastillenförmigen Granulats erlaubt und das die Nachteile, die die entsprechenden Verfahren nach dem Stand der Technik haben. nicht aufweist.

Demgemäß betrifft die vorliegende Erfindung ein Verfahren zur Herstellung eines pastillenförmigen Granulats aus einer Schmelze, wobei
(i) eine Schmelze bereitgestellt wird,
(ii) die bereitgestellte Schmelze zu Tropfen ausgeformt wird,
(iii) die Tropfen auf einer Kühlfläche abgelegt werden und
(iv) die abgelegten Tropfen zu dem Granulat erstarren,
das dadurch gekennzeichnet ist, daß die Schmelze einen Alkohol umfaßt und die Schmelze eine Schmelztemperatur von 30 °C und darüber aufweist.

Ebenso betrifft die vorliegende Erfindung das pastillenförmige Granulat, herstellbar durch ein Verfahren, in dem
(i) eine Schmelze bereitgestellt wird,
(ii) die bereitgestellte Schmelze zu Tropfen ausgeformt wird.
(iii) die Tropfen auf einer Kühlfläche abgelegt werden und
(iv) die abgelegten Tropfen zu dem Granulat erstarren,
das dadurch gekennzeichnet ist, daß die Schmelze einen Alkohol umfaßt und die Schmelze eine Schmelztemperatur von 30 °C und darüber aufweist.

Der Begriff "pastillenförmiges Granulat", wie er im Rahmen der vorliegenden Anmeldung verwendet wird, beschreibt ein im Umriß rundliches bis ovales Granulat. dessen untere Seite flach und dessen obere Seite flach bis konvex ist.

Hinsichtlich des Alkohols, den die Schmelze umfaßt, gibt es im allgemeinen keine Beschränkung, sofern die Schmelze, die den Alkohol umfaßt, eine Schmelztemperatur von 30 °C oder höher, bevorzugt eine Schmelztempteratur von 40 °C oder höher aufweist. Insbesondere kann der Alkohol ein linearer, verzweigter, cyclischer, gegebenenfalls substituierter Alkohol sein. Denkbar sind dabei sowohl Mono- als auch Polyole, die wiederum monomer, oligomer oder polymer sein können.

Selbstverständlich ist es auch denkbar, daß die Schmelze zwei oder mehr Alkohole umfaßt.

In einer bevorzugten Ausführungsform betrifft die vorliegenden Erfindung ein Verfahren, wie oben beschrieben, das dadurch gekennzeichnet ist, daß der Alkohol ausgewählt wird aus der Gruppe bestehend aus
(a) einem Alkohol (B), der durch ein Verfahren bereitgestellt wird. das die folgenden Stufen (I) bis (III) umfaßt:
   (I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
   (II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
   (III) Destillation des in Stufe (II) erhaltenen Gemisches (G-II) unter Erhalt des mindestens einen Alkohols (B) sowie eines Gemisches (G-III), umfassend das Lösungsmittel (L) und den Alkohol (A),
   wobei das in Stufe (III) erhaltene Lösungmittel (L) und der in Stufe (III) erhaltene Alkohol (A) als Mischung in Stufe (I) rückgeführt werden,
(b) einem Alkohol (C), der durch ein Verfahren bereitgestellt wird, das die folgenden Stufen (I) bis (III') umfaßt :
   (I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
   (II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
   (II') Hydrierung mindestens eines ungesättigten Alkohols (B) in dem aus Stufe (II) erhaltenen Gemisch (G-II) unter Erhalt eines Gemisches (G-II'), umfassend mindestens einen hydrierten Alkohol (C), den Alkohol (A) und das Lösungsmittel (L);
   (III') Destillation des in Stufe (II') erhaltenen Gemisches (G-II') unter Erhalt des mindestens einen Alkohols (C) sowie eines Gemisches (G-III'), umfassend das Lösungsmittel (L) und den Alkohol (A),
   wobei das in Stufe (III') erhaltene Lösungsmittel (L) und der in Stufe (III') erhaltene Alkohol (A) als Mischung in Stufe (I) rückgeführt werden, und
(c) einem Gemisch aus zwei oder mehr davon.

Bezüglich der Herstellung des Gemisches (G-I) in Stufe (I), das das Lösungsmittel (L), den Alkohol (A) und das Alkoholat (AL) umfaßt, existieren keine besonderen Beschränkungen. Es muß lediglich gewährleistet sein, daß (L) und (A) zusammen als Edukte zugegeben werden können.

In einer bevorzugten Ausführungsform wird die Stufe (I) in einer oder mehreren Destillationskolonnen durchgeführt, in der oder in denen das mindestens eine Alkoholat durch azeotrope Trocknung hergestellt wird. Hierbei ist es beispielsweise möglich, (L) und (A) bereits als Gemisch als einen einzigen Eduktstrom zuzuführen. Ebenso ist aber auch möglich, (L) und (A) getrennt zuzuführen und die Ströme erst in der mindestens einen Kolonne zu vereinen. Selbstverständlich ist es auch möglich, mehrere geeignete Lösungsmittel (L) und/oder mehrere geeignete Alkohole (A) einzusetzen. Hierbei ist es denkbar, diese in einem einzigen Eduktstrom der mindestens einen Kolonne zuzuführen oder in zwei oder mehreren Eduktströmen, die die jeweiligen Lösungsmittel (L) und/oder die jeweiligen Alkohole (A) umfassen.

Zusätzlich zu (L) und (A) wird als weiteres Edukt eine wäßrige Lösung eines oder mehrerer Alkali- und/oder Erdalkalihydroxide in einem oder mehreren Eduktströmen zugegeben. Ebenfalls ist in diesem Zusammenhang die Verwendung von Alkali- und/oder Erdalkalihydriden und/oder von Alkali- und/oder Erdalkalialkylverbindungen denkbar.

Sollte es im Rahmen der vorliegenden Erfindung erforderlich sein, können die einzelnen Eduktströme vor der Vermischung selbstverständlich auf eine gewünschte Temperatur gebracht werden. Eine solche Vortemperierung ist nach allen denkbaren Verfahren möglich.

Durch Abdestillieren von Wasser wird im Laufe der Durchführung der Stufe (I) aus dem mindestens einen Alkohol (A) und dem mindestens einen Alkaliund/oder Erdalkalihydroxid eine Mischung gewonnen, die mindestens ein Alkalioder Erdalkali-Alkoholat und das mindestens eine organische Lösungsmittel (L) umfaßt. Diese Mischung wird vorzugsweise aus dem Sumpf der verwendeten Kolonne gewonnen.

Im weiteren Verlauf der Durchführung der Stufe (I) wird aus dieser Mischung durch Destillation der überschüssige, mindestens eine Alkohol (A) abgetrennt. Diese Abtrennung kann sowohl in der gleichen Kolonne durchgeführt werden, in der das mindestens eine Alkoholat hergestellt wurde, als auch in einer oder mehreren nachgeschalteten Kolonnen. In einer bevorzugten Ausführungsform der vorliegenden Erfindung wird Stufe (I) in einer einzigen Kolonne durchgeführt, in der sowohl Wasser als auch der mindestens eine Alkohol (A) abdestilliert werden.

Im Rahmen der vorliegenden Erfindung wird soviel Alkohol (A) abgetrennt, daß das nach der Abtrennung von Wasser und der unvollständigen Abtrennung des mindestens einen Alkohols (A) resultierende Gemisch im allgemeinen Alkohol (A) im Bereich von 0 bis 55 Gew.-% enthält. Bevorzugt wird jedoch das Verfahren so geführt, daß der Alkohol (A) nicht quantitativ abgetrennt wird. Das Verfahren wird vielmehr so geführt, daß das nach der Abtrennung von Wasser und der unvollständigen Abtrennung des mindestens einen Alkohols (A) resultierende Gemisch Alkohol (A) im Bereich von 1 bis 55 Gew.-%, bevorzugt im Bereich von 2 bis 10 Gew.-% und besonders bevorzugt im Bereich ungefähr 5 Gew.-% enthält.

Bei der Abtrennung von Wasser in Stufe (I) ist es denkbar, je nach gewähltem Lösungsmittel (L) und Alkohol (A), daß ein bestimmter Anteil von (L) und/oder (A) zusammen mit dem Wasser als mehrphasiges Gemisch abdestilliert wird. Ist dies der Fall, so ist es im Rahmen des erfindungsgemäßen Verfahrens möglich. dieses abgetrennte, mehrphasige Gemisch, umfassend Wasser und (L) und/oder (A), einem Phasenscheider zuzuführen und die einzelnen Phasen zu trennen. Damit ist es möglich, solchermaßen abgetrenntes (L) und/oder (A) als Edukt(e) in Stufe (I) rückzuführen. Auch die solchermaßen abgetrennte wäßrige Phase kann im erfindungsgemäßen Verfahren, wie weiter unten beschrieben, in einer weiteren Stufe des Verfahrens eingesetzt werden.

Als Alkohole (A), die im erfindungsgemäßen Verfahren eingesetzt werden können, sind unter anderem primäre und sekundäre Alkohole mit 4 bis 8 Kohlenstoffatomen wie n-Butanol, Isobutanol, n-Pentanol, 3-Methylbutanol-1, 2-Methylbutanol-1, 2,2-Dimethylpropanol-1, n-Hexanol, 2-Ethylhexanol-1, Butanol-2, Pentanol-2, Pentanol-3, 2-Methylbutanol-3, 2-Methylbutanol-2 oder Cyclohexanol zu nennen. Ebenso sind natürlich auch Verbindungen der allgemeinen Struktur HO-CH₂-CH₂-OR''' denkbar, wobei R"' so gewählt wird, daß die jeweilige Verbindung in dem verwendeten Lösungsmittel(gemisch) löslich ist. Besonders bevorzugt sind hierbei n-Butanol und Isobutanol, insbesondere bevorzugt Isobutanol.

Als organische Lösungsmittel (L) werden im allgemeinen polare aprotische Lösungsmittel verwendet. Unter anderem kommen aliphatische, cycloaliphatische und/oder gegebenenfalls substituierte aromatische Kohlenwasserstoffe wie beispielsweise Cyclohexan, Benzol, Toluol, Xylol, Cumol oder p-Diisopropylbenzol, Acetale von Aldehyden und Ketonen, symmetrische oder asymmetrische Dialkylether von Ethan oder Butan oder Polyalkylenglykolen mit C₂- bis C₆-Alkylresten zum Einsatz. Besonders bevorzugt sind im Rahmen des erfindungsgemäßen Verfahrens aromatische Kohlenwasserstoffe, und hierbei besonders Xylol.

Als bevorzugte Hydroxide sind im Rahmen des erfindungsgemäßen Verfahrens die Alkalihydroxide zu nennen, wobei KOH besonders bevorzugt ist. Beim Einsatz der wäßrigen Lösung von KOH wird vorzugsweise ein Lösung verwendet. die im allgemeinen 2 bis 60 Gew.-%, bevorzugt 5 bis 50 Gew.-%, besonders bevorzugt 30 bis 35 Gew.-% KOH aufweist.

Das oben beschriebene Gemisch, das noch einen bestimmten Anteil an Alkohol (A) sowie Lösungsmittel (L) und Alkoholat (AL) umfaßt, wird im Anschluß an Stufe (I) der Stufe (II) zugeführt. In dieser Stufe (II) des erfindungsgemäßen Verfahrens wird mindestens eine Carbonylverbindung der allgemeinen Struktur R-CO-R' mit mindestens einem Alkin der allgemeinen Struktur R"-C≡C-H und dem in Stufe (I) erhaltenen Gemisch (G-I) umgesetzt und ein Alkohol (B) erhalten. In einer bevorzugten Ausführungsform des erfindungsgemäßen Verfahrens wird dazu in einem geeigneten Reakor das aus Stufe (I) erhaltene Gemisch (G-I), umfassend das Lösungsmittel (L), den Alkohol (A) und Alkoholat (AL), vorgelegt und das mindestens eine Alkin und die mindestens eine Carbonylverbindung eingeleitet.

Die Einleitung von Alkin und/oder Carbonylverbindung kann dabei nach allen geeigneten Methoden erfolgen. Möglich ist dabei beispielsweise, Alkin und Carbonylverbindung vor der Einleitung in den Reaktor zu einem Eduktstrom zusammenzuführen und in den Reaktor einzuleiten. Selbstverständlich ist es aber auch möglich, Alkin und Carbonylverbindung getrennt voneinander als einzelne Eduktströme in den Reaktor einzuleiten.

Weiterhin ist es im Falle, daß Alkin und Carbonylverbindung getrennt voneinander als einzelne Eduktströme in den Reaktor eingeleitet werden. möglich, zuerst Alkin einzuleiten und dann Carbonylverbindung. Natürlich ist es auch denkbar. zuerst Alkin einzuleiten und dann, bei anhaltender Einleitung des Alkins, die Carbonylverbindung zuzugeben und Alkin und Carbonylverbindung parallel einzuleiten. In einer bevorzugten Ausführungsform der Erfindung werden Alkin und Carbonylverbindung als getrennte Eduktströme in Stufe (II) gleichzeitig in das Gemisch (G-I) aus Stufe (I) eingeleitet. Das Einleiten kann dabei prinzipiell sowohl diskontinuierlich als auch kontinuierlich erfolgen. Vorzugsweise erfolgt das Einleiten kontinuierlich.

Je nach der gewählten Temperatur, bei der die Umsetzung in Stufe (II) erfolgen soll, kann es sinnvoll sein, die einzelnen Komponenten vor der Zuleitung in den Reaktor bereits auf die erforderliche Temperatur zu bringen, was nach allen Verfahren aus dem Stand der Technik denkbar ist. Insbesondere kann es erforderlich sein, das aus Stufe (I) erhaltene Gemisch, das aus der Kolonne abgeführt wird, vor der Zuleitung in den Reaktor der Stufe (II) abzukühlen.

Um eine möglichst gute Durchmischung von Alkin, Carbonylverbindung und dem in dem Gemisch aus Stufe (I) enthaltenen, mindestens einen Alkoholat zu erreichen, wird die Reaktionsmischung in Stufe (II) gerührt. Diese Rührung kann prinzipiell nach allen gängigen Verfahren aus dem Stand der Technik erfolgen. Denkbar ist aber auch, durch eine spezielle Art der Einleitung den Durchmischungsvorgang entweder komplett vorzunehmen oder die Rührung damit zu unterstützen.

In einer bevorzugten Ausführungsform des erfindungsgemäßen Verfahren wird die Reaktionsmischung in Stufe (II) in einer Mischvorrichtung gemischt, wie sie beispielsweise in der DE-C 42 20 239, die diesbezüglich vollumfänglich durch Bezugnahme in den Kontext der vorliegenden Anmeldung einbezogen wird. beschrieben ist. Sämtliche geeignete Ausführungsformen der dort beschriebenen Mischvorrichtung sind denkbar. So können beispielsweise Alkin, Carbonylverbindung und das Gemisch aus Stufe (I) in separaten Strömen in die Mischvorrichtung eingebracht werden, die dementsprechend mindestens 3 Einlaßöffnungen aufweisen muß. Ebenso können (a) Alkin und Carvonylverbindung oder (b) Alkin und Gemisch aus Stufe (I) vor dem Einbringen in die Mischvorrichtung gemischt werden, beispielsweise unter Verwendung einer Mischvorrichtung der hier beschriebenen Art, und das resultierende Gemisch als ein Strom und (a) Gemisch aus Stufe (I) oder (b) Carbonylverbindung oder (c) Alkin als weiterer Strom in die Mischvorrichtung eingebracht werden. Dementsprechend muß die Mischvorrichtung in diesem Fall mindestens zwei Einlaßöffnungen aufweisen. Für jede der Ausführungsformen ist es denkbar, daß die einzelnen Ströme, die in die Mischvorrichtung eingebracht werden sollen, vor dem Einbringen durch eine geeignete Vorrichtung, die der Mischvorrichtung vorgeschaltet sind ist, jeweils in zwei oder mehr Ströme aufgeteilt werden, die anschließend in die Mischvorrichtung eingebracht werden.

Weiter können zur Mischung der einzelnen Komponenten, die in Stufe (II) zur Reaktion gebracht werden sollen, auch zwei oder mehr Mischvorrichtungen ider hier beschriebenen Art eingesetzt werden. Beispielsweise können in jeder dieser mindestens zwei Mischvorrichtungen Mischungen hergestellt werden. die anschließend vereinigt werden.

Sollen die Komponenten, die in Stufe (II) zur Reaktion gebracht werden. mit Hilfe dieser Mischvorrichtung lediglich gemischt werden, so ist im Rahmen der vorliegenden Erfindung darauf zu achten, daß die Reaktionsbedingungen in der Mischvorrichtung, wie beispielsweise Temperatur und Druck, derart gewählt werden. daß keine unerwünschten Reaktionen beim Mischvorgang erfolgen.

Bevorzugt wird die Mischvorrichtung als Reaktionsmischpumpe eingesetzt. In der Mischvorrichtung werden die einzelnen Komponenten, die in Stufe (II) zur Reaktion gebracht werden, sowohl gemischt als auch miteinander zur Reaktion gebracht, wie dies untenstehend beschrieben ist.

Generell ist es im Rahmen der vorliegenden Erfindung denkbar. zur. Umsetzung von Alkin mit Carbonylverbindung und dem aus der Stufe (I) erhaltenen Gemisch in Stufe (II) zu dem mindestens einen organischen Lösungsmittel (L) und dem mindestens einen Alkohol (A), die in dem aus Stufe (I) erhaltenen Gemisch enthalten sind, zusätzlich ein oder mehrere weitere geeignete Lösungsmittel zuzugeben.

Als Carbonylverbindungen der allgemeinen Struktur R-CO-R' werden im erfindungsgemäßen Verfahren beispielsweise aliphatische, araliphatische oder heterocyclische Ketone mit bis zu 30 C-Atomen eingesetzt. Dabei könne R und R' gleich oder verschieden sein und entweder zwei getrennte Reste oder aber verbrückt sein. Ebenso ist es möglich, daß die Reste R und/oder R' olefinische oder acetylenische Funktionen aufweisen. Beispielhaft seien Aceton. Isobutylmethylketon 6,10-Dimethyl-5-undecen-2-on, 6,11,14-Trimethyl-2-Pentadecanon, 2-Methyl-2-hepten-6-on, 2-Methylheptan-6-on, [4(2,6,6-Trimethyl-1--cyclohexenyl)-3-buten-2-on], Methylethylketon, Cyclohexanon, Acetophenon, Benzophenon, Piperidon-4 genannt, wobei Aceton, Isobutylmethylketon, 6,10-Dimethyl-5-undecen-2-on, 6,11,14-Trimethyl-2-Pentadecanon, 2-Methyl-2-hepten-6-on, 2-Methylheptan-6-on und [4(2,6,6-Trimethyl-1-cyclohexenyl)-3-buten-2-on] bevorzugt sind.

Auch der Einsatz von Aldehyden, die keinen aciden Wasserstoff aufweisen, ist prinzipiell denkbar. Hierbei sind unter anderem Aldehyde RCHO zu nennen, wobei R = H oder ein Alkylrest mit bis zu 30 C-Atomen ist. Als bevorzugt verwendeter Aldehyd ist CH₂O zu nennen.

Als Alkine der allgemeinen Struktur R"-C≡C-H seien solche genannt, bei denen R" ausgewählt wird aus der Gruppe bestehend aus Wasserstoff und einem aliphatischen, araliphatischen oder aromatischen Rest mit bis zu 15 C-Atomen. Als Beispiele seien etwa Acetylen, Propin, 1-Butin, 1-Pentin. 1-Hexin. Phenylacetylen. Benzylacetylen, 3-Methyl-1-butin oder Verbindungen wie etwa 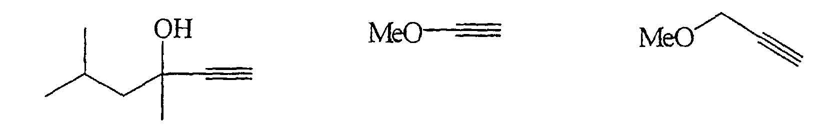 genannt.

Wird im erfindungsgemäßen Verfahren Acetylen als Alkin eingesetzt, so ist es denkbar, durch Umsetzung mit entsprechenden Mengen an Carbonylverbindung und Alkoholat sowohl Alkin-Monoole als auch Alkin-Diole herzustellen. Bei Einsatz eines Alkins, bei dem R" ≠ H, werden Alkin-Monoole erzeugt.

Selbstverständlich ist es im Rahmen des erfindungsgemäßen Verfahrens auch denkbar, als Alkin der allgemeinen Struktur R"-C≡C-H ein Alkin-Monool einzusetzen und durch Umsetzung mit der Carbonylverbindung und dem Alkoholat daraus ein Alkin-Diol herzustellen.

Bevorzugt wird als Alkin der allgemeinen Struktur R"-C≡C-H im erfindungsgemäßen Verfahren Acetylen verwendet. Aus den oben als bevorzugt genannten Carbonylverbindungen sind damit u.a. die folgenden Alkinole herstellbar, wobei der Begriff "Alkinol" prinzipiell alle Verbindungen umfaßt, die sowohl mindestens eine C-C-Dreifachbindung als auch eine oder mehrere Hydroxylgruppen aufweisen:
2,5-Dimethylhex-3-in-2,5-diol (DMHDY), 3-Methyl-3-hydroxy-but-1-in, 2,4,7,9-Tetramethyl-4,7-dihydroxy-dec-5-in, 3,7,11-Trimethyl-6-dodecan-1-in-3-ol, 3,7,11,15-Tetramethyl-1-hexadecin-3-ol. 3,7-Dimethyl-oct-1-in-6-en-3-ol, 3,7-Dimethyl-oct-1-in-3-ol, 1-Penten-4-in-3-ol, Propinol, But-2-in-1-ol, But-1-in-3-ol. oder die Verbindungen

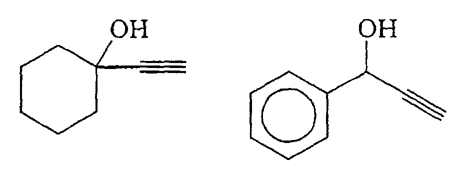

Bei der Herstellung des ungesättigten Alkohols kann das stöchiometrische Verhältnis von Carbonylverbindung zu Alkin im wesentlichen beliebig gewählt werden.

Werden beispielsweise Alkindiole aus Carbonylverbindung und Acetylen hergestellt, wird bevorzugt ein stöchiometrisches Verhältnis Carbonylverbindung: Acetylen im Bereich von 1,9 : 1 bis 2,1 : 1, besonders bevorzugt von etwa 2 : 1 gewählt. Das stöchiometrische Verhältnis Alkoholat : Carbonylverbindung liegt bevorzugt im Bereich von 0,9 : 1 bis 2,1 : 1 , besonders bevorzugt im Bereich von 1 : 1 bis 1,5: 1 und insbesondere bei etwa 1,1 : 1. Werden beispielsweise Alkinmonoole aus Carbonylverbindung und Acetylen hergestellt, wird bevorzugt ein stöchiometrisches Verhältnis Carbonylverbindung : Acetylen im Bereich von 1 : bis 0,5 : 1, besonders bevorzugt von etwa 0,6 : 1 gewählt. Das stöchiometrische Verhältnis Alkoholat : Carbonylverbindung liegt bevorzugt im Bereich von 1 : I bis 0,2 : 1 und insbesondere bei etwa 0,3 : 1.

Die Reaktionstemperatur bei der Umsetzung entsprechender Edukte zu Alkindiolen liegt im Rahmen des erfindungsmäßigen Verfahrens bevorzugt im Bereich von 0 bis 50 °C, weiter bevorzugt im Bereich 10 bis 40 °C und insbesondere bevorzugt im Bereich von 25 bis 35 °C.

Die Reaktionstemperatur bei der Umsetzung entsprechender Edukte zu Alkinmonoolen liegt im Rahmen des erfindungsmäßigen Verfahrens bevorzugt im Bereich 0 bis 50 °C, weiter bevorzugt im Bereich von 0 bis 35 °C und insbesondere bevorzugt im Bereich von 0 bis 20 °C.

Die Drücke bei den genannten Umsetzungen liegen bei der Herstellung von Alkinmonoolen und Alkindiolen im Rahmen des erfindungsmäßigen Verfahrens bevorzugt im Bereich von 1 bis 20 bar, weiter bevorzugt im Bereich von 1 bis 5 bar und insbesondere bei 1 bar.

Die Ausbeute an ungesättigten Alkohol (B), die im Rahmen des erfindungsgemäßen Verfahrens in Stufe (II) erhalten wird. liegt bevorzugt bei mindestens 75 %. weiter bevorzugt bei mindestens 80 %, besonders bevorzugt bei mindestens 85 % und insbesondere bevorzugt bei mindestens 90 %.

Wie bereits oben beschrieben, wird die Umsetzung gemäß Stufe (II) vorzugsweise in einer Reaktionsmischpumpe durchgeführt, wie sie in der DE-C 42 20 239 beschrieben ist, wobei die Ausführungsform der Reaktionsmischpumpe nicht auf die dort offenbarte Mischvorrichtung beschränkt ist. Sämtliche denkbaren Ausführungsformen einer Vorrichtung, in der die Edukte der Stufe (II) sowohl gemischt als auch zur Reaktion gebracht werden können, sind ebenso denkbar.

Letztendlich erhält man in dieser Stufe (II) ein Gemisch (G-II), das mindestens einen ungesättigten Alkohol (B) sowie (A) und (L) umfaßt.

Weiter werden im erfindungsgemäßen Verfahren bevorzugt Alkohole, die von der Schmelze umfaßt werden, durch ein Verfahren hergestellt, das die Stufen (I), (II), (II') und (III') umfaßt.

Da in Stufe (II) ein Alkin-Monool und/oder ein Alkin-Diol, je nach Wahl der Reaktionspartner, gebildet wird, sind im Rahmen des vorliegenden Verfahrens verschiedene Hydrierungen in Stufe (II') denkbar. Wurde beispielsweise ein Alkin-Monool oder ein Alkin-Diol erhalten, so kann das Alkin-Monool oder Alkin-Diol in einer partiellen Hydrierung zum entsprechenden Alkenol hydriert werden. Es ist aber auch möglich, durch entsprechende Wahl der Hydrierbedingungen das jeweilige Alkanol herzustellen.

Die jeweilige Hydrierung kann hierbei prinzipiell nach allen geeigneten Verfahren gemäß dem Stand der Technik durchgeführt werden. So kann die jeweilige Hydrierung in einem Reaktor oder aber in mehreren hintereinander geschalteten Reaktoren durchgerührt werden. Jeder Reaktor kann dabei in allen denkbaren Fahrweisen betrieben werden, wobei vor allem Riesel- und Sumpffahrweise im Festbettreaktor genannt sein sollen. In einer bevorzugten Ausrührungsform findet die Hydrierung in zwei hintereinander geschalteten Rohrreaktoren (Festbett) statt, von denen der erste rückvermischt in Rieselfahrweise, der zweite im geraden Durchgang in Riesel- oder Sumpffahrweise betrieben wird.

Ebenso kann die Hydrierung auch in zwei oder mehr parallel geschalteten Reaktionen durchgeführt werden.

Hierbei ist es möglich, daß die Komponenten, die in den einen Hydrierreaktor oder in die mehreren Hydrierreaktoren eingeleitet werden, vor der Einleitung vorgeheizt oder auch vorgekühlt werden. Dies kann beispielsweise in einem oder mehreren Wärmetauschern erfolgen.

Weiter ist es denkbar, die Temperatur des Hydrierreaktors oder der Hydrierreaktoren selbst zu regeln, was nach allen Verfahren nach dem Stand der Technik durchgeführt werden kann. Dadurch ist es beispielsweise möglich, eine nachlassende Hydrieraktivität des mindestens einen, zur Hydrierung eingesetzten Katalysators, wie unten beschrieben, durch Erhöhung der Reaktortemperatur zu kompensieren. Diese Erhöhung der Reaktortemperatur kann beispielsweise dadurch erfolgen, daß bei einer exothermen Hydrierungsreaktion die Kühlung des Reaktors vermindert wird. Ebenso ist es natürlich denkbar, die Reaktortemperatur aktiv durch Erwärmung von außen zu erhöhen.

Wird im erfindungsgemäßen Verfahren ein Alkinol zu einem Alkenol hydriert, so können hierfür sämtliche geeigneten, aus dem Stand der Technik bekannten Katalysatoren eingesetzt werden. Als mögliche Katalysatoren sind hierbei unter anderem etwa Pd oder Pd/Pb auf CaCO₃ (Lindlar-Katalysator) zu nennen. Gegebenenfalls werden die Katalysatoren wie beispielsweise Pd zur Erzielung guter Selektivitäten z.B. durch CO partiell vergiftet.

Wird im erfindungsgemäßen Verfahren ein Alkinol zu einem Alkanol hydriert, so können hierfür ebenfalls sämtliche geeigneten, aus dem Stand der Technik bekannten Katalysatoren verwendet werden. Bekannte Katalysatoren sind beispielsweise Pd-, Pt-, Ni- (auch Raney-Nickel), Co-, Ru- oder Rh-Katalysatoren, wobei diese geträgert oder als Vollkontaktkatalysatoren eingesetzt werden können. Als Träger können hierbei alle geeigneten gängigen Träger wie beispielsweise Al₂O₃, SiO₂ oder C verwendet werden.

In einer bevorzugten Ausführungsform des Verfahrens, in der ein oder mehrere Alkinole zu den entsprechenden Alkanolen hydriert werden, werden als Katalysatoren geträgerte Katalysatoren oder Vollkontakte verwendet. Als aktives Hydriermetall sind hierbei vor allem die Metalle der 1., 7. und 8. Nebengruppe des Periodensystems zu nennen. Vorzugsweise werden dabei Ni, Ru, Pd, Pt und Rh eingesetzt.

In einer besonders bevorzugten Ausführungsform bei der Hydrierung von Alkinolen zu Alkanolen wird im Rahmen des erfindungsgemäßen Verfahrens als Katalysator ein geträgerter Pd-Katalysator verwendet, wobei das Trägermaterial Aluminiumoxid umfaßt.

Die Hydrierung der Alkinole zu den jeweiligen Alkanolen findet im erfindungsgemäßen Verfahren bei Drücken von im allgemeinen 1 bis 300 bar, bevorzugt 10 bis 200 bar, besonders bevorzugt 15 bis 100 bar und insbesondere bevorzugt 20 bis 50 bar statt.

Im allgemeinen liegen die Temperaturen bei der Hydrierung im erfindungsgemäßen Verfahren bei der Hydrierung von Alkinolen zu den jeweiligen Alkanolen im Bereich von 30 bis 250 °C, bevorzugt im Bereich von 50 bis 200 °C und besonders bevorzugt im Bereich von 80 bis 160 °C.

Werden im erfindungsgemäßen Verfahren Alkinole zu den jeweiligen Alkenolen hydriert, so liegen die Temperaturen bei der Hydrierung im allgemeinen im Bereich von 30 bis 200 °C, bevorzugt im Bereich von 40 bis 150 °C und besonders bevorzugt im Bereich von 50 bis 130 °C.

Werden im erfindungsgemäßen Verfahren zwei oder mehr hintereinander geschaltete Reaktoren bei der Hydrierung eingesetzt, so ist es denkbar, daß in den einzelnen Reaktoren verschiedene Drucke und/oder verschiedene Temperaturen zur Hydrierung eingestellt werden. So ist es unter anderem möglich, die Temperatur eines Produktstroms aus einem ersten Reaktor, der aufgrund freiwerdender Reaktionswärme eine höhere Temperatur aufweist als der Eduktstrom in diesen ersten Reaktor, vor dem Einleiten in einen zweiten Reaktor nicht zu regeln. Natürlich ist es aber auch möglich, beispielsweise über eine zwischen diesen beiden Reaktoren vorgesehene Zwischenkühlung den Produktstrom aus dem ersten Reaktor vor dem Einleiten in den zweiten Reaktor abzukühlen. So ist es beispielsweise möglich, die Hydriertemperatur im zweiten Reaktor individuell an die Aktivität des dort verwendeten Katalysators anzupassen.

Selbstverständlich ist es auch denkbar, aus dem Alkinol eine Mischung aus Alkenol und Alkanol herzustellen. Dies ist beispielsweise durch entsprechende Wahl der Hydrierbedingungen möglich. Eine weitere Möglichkeit hierfür besteht darin, den Produktstrom aus Stufe (II), der aus dem in Stufe (II) erhaltenen Gemisch besteht, in zwei oder mehr Ströme zu teilen und jeden Strom in separaten Reaktoren zu hydrieren, wobei dann die Hydrierbedingungen in jedem Reaktor unterschiedlich sein können und somit auf einfache Art und Weise Alkenole und Alkanole aus dem Alkinol hergestellt werden können.

Je nach Hydrierbedingungen können aus den bereits oben beschriebenen, bevorzugt hergestellten Alkinolen folgende Alkenole und/oder Alkanole hergestellt werden:
2,5-Dimethylhexan-2,5-diol (DMHD), 3-Methyl-3-hydroxy-but-1-en, 2-Methyl-2-hydroxy-butan, 3,7,11-Trimethyl-3-hydroxy-1,6-dodecadien, 3,7,11,15-Tetramethyl-hexadec-1-en-3-ol, 3,7-Dimethyl-1,6-octadien-3-ol, 3,7-Dimethyl-oct-1-en-3-ol, 3-Methyl-1-(2,6,6-Trimethyl-1-cyclohexen-1-ol), 1,4-Pentadien-3-ol, 3-Methyl-1-(2,6,6-trimethyl-1-cyclohexen-1-ol).

Die Stufe (II') ist nicht auf eine Hydrierung der Dreifachbindung des aus der Stufe (II) erhaltenen, mindestens einen Alkinols beschränkt. Je nach der chemischen Struktur des in Stufe (II) eingesetzten mindestens einen Alkins und/oder der mindestens einen Carbonylverbindung ist es denkbar, daß in Stufe (II') die Reste R und/oder R' und/oder R" chemisch modifiziert werden, indem z.B. Reaktionen an in diesen Resten enthaltenen funktionellen Gruppen durchgeführt werden. Weiter ist es im Rahmen des erfindungsgemäßen Verfahrens auch denkbar, daß solche Umsetzungen zusätzlich zu den weiter oben beschriebenen Hydrierungen durchgeführt werden.

In einer weiter bevorzugten Ausführungsform des erfindungsgemäßen Verfahrens wird die Stufe (II), wie oben beschrieben, in Einzelschritten (aa) bis (ff) durchgerührt. Demgemäß beschreibt die vorliegende Erfindung auch ein Verfahren, wie oben beschrieben, in dem die Stufe (II) die folgenden Schritte (aa) bis (ff) umfaßt:
(aa) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt einer Mischung (M-aa);
(bb) Hydrolyse der aus Schritt (aa) erhaltenen Mischung (M-aa) unter Erhalt einer mehrphasigen Mischung (M-bb), umfassend mindestens eine organische Phase;
(cc) Abtrennung der mindestens einen organischen Phase aus der in Schritt (bb) erhaltenen mehrphasigen Mischung (M-bb);
(dd) Extraktion der mindestens einen in Schritt (cc) abgetrennten organischen Phase;
(ee) Neutralisation der mindestens einen, in Schritt (dd) extrahierten organischen Phase unter Erhalt einer Mischung (M-ee), umfassend mindestens ein Alkali- oder Erdalkalisalz;
(ff) Abtrennung des mindestens einen Alkali- oder Erdalkalisalzes aus der in Schritt (ee) erhaltenen Mischung (M-ee) unter Erhalt eines Gemisches (G-II), umfassend mindestens den Alkohol (A), das Lösungsmittel (L) und einen ungesättigten Alkohol (B).

Aus der Umsetzung der mindestens einen Carbonlyverbindung mit dem mindestens einen Alkin und dem in Stufe (I) erhaltenen Gemisch (G-I), wie oben beschrieben, resultiert zunächst eine Mischung (M-aa), die dem Schritt (bb) zugeführt wird. In diesem Schritt (bb) wird der mindestens eine ungesättigte Alkohol (B) durch Hydrolyse freigesetzt.

Für die Art und Weise der Durchführung der Hydrolyse bestehen prinzipiell keine Beschränkungen. So kann diese beispielsweise in einem oder mehreren Reaktoren durchgerührt werden, wobei die Temperatur im jeweiligen Reaktor gegebenenfalls geregelt werden kann. Denkbar ist beispielsweise eine Kühlung durch Sole. Ebenso denkbar ist auch eine Kühlung des Hydrolysewassers, die ebenfalls nach allen denkbaren Verfahren gemäß dem Stand der Technik durchgeführt werden kann. Im allgemeinen wird bei der Durchführung der Hydrolyse die Reaktionsmischung gerührt, wobei dies nach allen gängigen Verfahren nach dem Stand der Technik erfolgen kann. Beispielsweise kann zum Rühren und Mischen eine Mischvorrichtung, wie sie obenstehend beschrieben ist und beispielsweise in der DE-C 42 20 239 offenbart ist, eingesetzt werden.

Die wäßrige Phase, die zu diesem hydrolytischen Schritt (bb) eingesetzt wird, resultiert in einer besonders bevorzugten Ausführungsform des erfindungsgemäßen Verfahrens aus der Extraktion in Schritt (dd). Selbstverständlich kann daneben noch weiteres Wasser zugesetzt werden. Sowohl das zur Hydrolyse verwendete Wasser als auch die zu hydrolisierende Reaktionsmischung können vor der Einleitung in den mindestens einen Hydrolysereaktor auf die gewünschte Temperatur gebracht werden, was beispielsweise durch Wärmetauscher erfolgen kann.

Aus der Hydrolyse in Schritt (bb) wird eine Mischung (M-bb) erhalten, die aus mindestens zwei Phasen besteht, aus mindestens einer organischer Phase und mindestens einer wäßrigen Phase. Aus dieser Mischung (M-bb) wird in Schritt (cc) die mindestens eine organische Phase abgetrennt. Diese Phasentrennung kann prinzipiell nach allen gängigen Methoden erfolgen. Dabei ist es denkbar, daß bei Vorliegen von zwei oder mehr organischen Phasen diese zusammen als mehrphasige organische Mischung abgetrennt werden oder als zwei oder mehr separate organische Phasen. Ebenfalls denkbar ist es, bei Vorliegen von zwei oder mehr wäßrigen Phasen diese zusammen als mehrphasige wäßrige Mischung abzutrennen oder als zwei oder mehr separate wäßrige Phasen.

In einer bevorzugten Ausführungsform der vorliegenden Erfindung wird die hydrolysierte Mischung (M-bb) aus Schritt (bb) in ein oder mehrere Phasentrenngefäße geleitet, die so ausgelegt sein können, daß die Temperatur der aufzutrennenden mehrphasigen Mischung im Trenngefäß regelbar ist. Vorzugsweise findet die Phasentrennung bei Temperaturen statt, die im allgemeinen im Bereich von 10 bis 80 °C, bevorzugt im Bereich von 20 bis 60 °C und besonders bevorzugt im Bereich von 40 °C liegen.

In einer besonders bevorzugten Ausführungsform des erfindungsgemäßen Verfahrens wird die mindestens eine wäßrige Phase, die einen Gehalt an Alkali- und/oder Erdalkalihydroxid im Bereich von 2 bis 60 Gew.-%, bevorzugt im Bereich von 30 bis 35 Gew.-% aufweist, als Edukt in Stufe (I) rückgeführt. Um bei der in Stufe (I) erfolgenden Alkoholatherstellung eine Basenkonzentration zu erreichen, die die Herstellung des mindestens eines Alkoholates (AL) ermöglicht, kann es notwendig sein, daß in Stufe (I), zusätzlich zu der aus Schritt (cc) erhaltenen wäßrigen, Alkali- und/oder Erdalkalihydroxid umfassenden mindestens einen wäßrigen Phase, eine weitere wäßrige Lösung, die Alkali- und/oder Erdalkalihydoxid umfaßt, zugesetzt wird.

Die mindestens eine organische Phase wird in einem nächsten Schritt (dd) extrahiert. Wurde in Schritt (cc) eine einzige organische Phase oder eine mehrere organische Phasen umfassende Mischung abgetrennt, so wird diese Phase oder diese Mischung extrahiert. Wurden mehrere organische Phasen abgetrennt, so ist es beispielsweise denkbar, jede einzelne organische Phase separat zu extrahieren.

In einer bevorzugten Ausführungsform der vorliegenden Erfindung wird zur Extraktion die wäßrige Phase eingesetzt, die aus der Stufe (I) durch Destillation erhalten wird. Dabei ist es natürlich denkbar, daß dieser wäßrigen Phase zusätzliches Wasser zugesetzt wird.

Die Extraktion kann nach allen möglichen Verfahren, die aus dem Stand der Technik bekannt sind, durchgeführt werden. Hierbei ist unter anderem die Verwendung von Kolonnen wie Siebboden-Kolonnen, pulsierende Hebboden-Kolonnen oder Füllkörperkolonnen zu nennen. In einer bevorzugten Ausrührungsform wird die Extraktion im Gegenstromverfahren durchgeführt, wobei die Temperatur des Extraktionsgutes bei der Extraktion im allgemeinen im Bereich von 30 bis 50 °C, bevorzugt bei ungefähr 40 °C liegt.

In diesem Extraktionsschritt (dd) wird der mindestens einen organischen Phase das mindestens eine Alkali- oder Erdalkalihydroxid soweit entzogen, daß der Gehalt der mindestens einen organischen Phase an Alkali- und/oder Erdalkalihydroxid weniger als 1 Gew.-%, bevorzugt weniger als 0,1 Gew.-% und besonders bevorzugt weniger als 0,01 Gew.-% beträgt.

Die wäßrige Phase, die aus der Extraktion resultiert, wird in einer bevorzugten Ausführungsform des vorliegenden erfindungsgemäßen Verfahrens in Schritt (bb) rückgeführt.

Die mindestens eine extrahierte organische Phase kann in dem weiteren Schritt (ee) neutralisiert werden. Die Neutralisation erfolgt hierbei durch Zusatz von Säure, wobei als Säuren u.a. Phosphorsäure, Ameisensäure, Essigsäure, Schwefelsäure oder Kohlensäure zu nennen sind. Ebenso denkbar ist die Verwendung von festem Kohlendioxid. Bevorzugt wird im erfindungsgemäßen Verfahren Phosphorsäure eingesetzt.

In dem folgenden Schritt (ff) wird das bei der Neutralisation entstandene, mindestens eine Alkali- oder Erdalkalisalz aus der aus Schritt (ee) erhaltenen Mischung (M-ee) abgetrennt. Diese Abtrennung kann generell nach allen aus dem Stand der Technik bekannten Verfahren erfolgen. Die Salzabtrennung kann im erfindungsgemäßen Verfahren unterbleiben, wenn das Produkt, von dem Salz abgetrennt wird, thermisch relativ wenig belastet wird. Dies ist beispielsweise dann der Fall, wenn der mindestens eine ungesättigte Alkohol (B) nicht hydriert wird.

In einer bevorzugten Ausführungsform des erfindungsgemäßen Verfahrens erfolgt die Abtrennung des mindestens einen Alkali- oder Erdalkali-Salzes aus der mindestens einen in Schritt (ee) erhaltenen Mischung (M-ee) über Totalverdampfung der organischen Substanzen durch Verwendung beispielsweise eines Dünnschichtverdampfers oder eines Fallfilmverdampfers. Das mindestens eine zurückbleibende Alkali- oder Erdalkali-Salz wird mittels einer oder mehrerer geeigneter Substanzen aus dem Verdampfer ausgespült. Als hierfür geeignete Substanzen seien unter anderem Polyalkylenglykole wie etwa Polyethylenglykol genannt. In einer besonders bevorzugten Ausrührungsform wird von den Substanzen, die zum Ausspülen des mindestens einen Alkali- oder Erdalkali-Salzes eingesetzt wird, ein Anteil von mindestens 0,5 %, bevorzugt 1 bis 2 % und besonders bevorzugt mindestens 1 bis 10% rückgeführt.

Sollte der mindestens eine ungesättigte Alkohol (B) im erfindungsgemäßen Verfahren nicht hydriert werden oder ist der mindestens eine hydrierte Alkohol (C) nicht ausreichend thermostabil, so daß bei den bei der Totalverdampfung auftretenden Temperaturen ein Verlust an Wertprodukt auftritt, so kann die Salzabtrennung auch über Ionentausch erfolgen. Bezüglich des Ionentausches sind alle geeigneten Verfahren, die aus dem Stand der Technik bekannt sind, denkbar.

In einer weiter bevorzugten Ausführungsform wird zusätzlich zu der aus Schritt (ee) erhaltenen Mischung (M-ee) derjenige Hochsiedersumpf in den Verdampfer geleitet, der bei der Destillation in Stufe (III) anfällt. Damit wird erreicht, daß Reste an Wertprodukt, die in dem Hochsiedersumpf enthalten sind, in den Prozeß zurückgeführt werden. Ein weiterer Vorteil, der mit dieser Rückführung verbunden ist, ist die Tatsache, daß der genannte Hochsiedersumpf als Schmiermittel für den Verdampfer wirkt.

Das Verdampferdestillat, das aus der Totalverdampfung der organischen Substanzen resultiert, wird im erfindungsgemäßen Verfahren kondensiert. In einer bevorzugten Ausführungsform des erfindungsgemäßen Verfahrens erfolgt diese Kondensation in mindestens zwei, in einer weiter bevorzugten Ausführungsform in zwei Schritten. Die Temperatur, bei der der erste Kondensationsschritt erfolgt, liegt im allgemeinen im Bereich von 30 bis 80 °C, bevorzugt im Bereich von 35 bis 50 °C und weiter bevorzugt bei etwa 40 °C. Die Temperatur, bei der der zweite Kondensationsschritt erfolgt, liegt im allgemeinen im Bereich von 0 bis 40 °C, bevorzugt im Bereich von 5 bis 20 °C und weiter bevorzugt bei etwa 10 °C.

Das Gemisch (G-II), das nach den Kondensationsschritten anfällt und das den mindestens einen ungesättigten Alkohol (B), (L) und (A) umfaßt, wird dann der Stufe (II') oder (III) zugeführt. Dabei ist es denkbar, daß, im Falle mehrerer Kondensationsschritte, nur das Kondensat eines Kondensationsschrittes in Stufe (II') und/oder (III) weiterverarbeitet wird. Es ist aber auch möglich, daß mehrere Kondensatströme zusammen in Stufe (II') und/oder (III) weiterverarbeitet werden.

In Stufe (III) oder (III') der vorliegenden Erfindung wird das aus der vorhergehenden Stufe erhaltene Gemisch (G-II) oder (G-II') destilliert und bei dieser Destillation der mindestens eine, in der vorhergehenden Stufe hergestellte Alkohol (B) oder (C) erhalten. Ebenso werden auch das mindestens eine organische Lösungsmittel (L) und der mindestens eine Alkohol (A) abgetrennt. Diese Destillation kann wiederum nach allen gängigen Verfahren, die aus dem Stand der Technik bekannt sind, erfolgen. In einer bevorzugten Ausführungsform des erfindungsgemäßen Verfahrens wird die Destillation in einer Packungskolonne mit Trennwand durchgeführt.

Die nach der Destillation in Stufe (III) oder (III') erhaltenen (L) und (A) werden nach dem erfindungsgemäßen Verfahren als Edukte in die Stufe (I) rückgeführt, wobei (L) und (A) getrennt voneinander oder als Mischung in Stufe (I) rückgeführt werden können.

Jede der Stufen (I), (II), (II'), (III) und (III') sowie jeder der Schritte (aa) bis (ff) kann prinzipiell kontinuierlich oder diskontinuierlich durchgeführt werden. In einer bevorzugten Ausführungsform des erfindungsgemäßen Verfahrens sind diese Stufen und Schritte so konzipiert, daß jede Stufe und jeder Schritt kontinuierlich durchgeführt werden kann.

In einer bevorzugten Ausführungsform des erfindungsgemäßen Verfahrens fällt in Stufe (III) oder Stufe (III') der mindestens eine ungesättigte Alkohol (B) oder der mindestens eine Alkohol (C) im Zuge der Destillation als schmelzflüssiges Produkt an. Dieses kann im Anschluß an die Destillation direkt zu Tropfen ausgeformt werden. Das Ausformen der Tropfen an sich stellt naturgemäß einen diskontinuierlichen Prozeß dar. Da die Zufuhr von Schmelze zur Tropfenausformung bevorzugt kontinuierlich erfolgt und die ausgeformten Tropfen, die auf der Kühlfläche abgelegt werden, von der Kühlfläche als pastillenförmiges Granulat bevorzugt kontinuierlich abtransportiert werden, ist aber der gesamte Verfahrenablauf bevorzugt ein kontinuierlicher Prozeß.

Demgemäß beschreibt die vorliegende Erfindung auch ein Verfahren, wie oben beschrieben, in dem jede Stufe und jeder Schritt als kontinuierlicher Prozeß erfolgt.

Die vorliegende Erfindung beschreibt insbesondere auch ein integriertes Verfahren zur Herstellung eines schmelzflüssigen ungesättigten Alkohols (B) und Herstellung eines pastillenförmigen Granulats aus dieser Schmelze, das die folgenden. kontinuierlich durchgeführten Stufen (aaa) bis (jjj) umfaßt:
(aaa) Umsetzung mindestens eines Alkali- oder Erdalkalihydroxids mit mindestens einem Alkohol (A) in mindestens einem organischen Lösungsmittel (L) unter Verwendung der in Stufe (ddd) erhaltenen wäßrigen Phase (P-ddd) und unter Erhalt eines Gemisches (G-aaa), umfassend mindestens das Lösungsmittel (L), den Alkohol (A) und ein Alkoholat (AL), und unter Erhalt einer wäßrigen Phase (P-aaa), die der Stufe (eee) zugeführt wird;
(bbb) Umsetzung mindestens einer Carbonylverbindung der allgemeinen Struktur R-CO-R' mit mindestens einem Alkin der allgemeinen Struktur R"-C≡C-H und dem in Stufe (aaa) erhaltenen Gemisch (G-aaa) unter Erhalt eines Gemisches (G-bbb), umfassend mindestens einen ungesättigten Alkohol (B);
(ccc) Hydrolyse des Gemisches (G-bbb) aus Stufe (bbb) unter Verwendung der in Stufe (eee) erhaltenen wäßrigen Phase (P-eee) unter Erhalt einer mehrphasigen Mischung (M-ccc), umfassend mindestens eine organische Phase und mindestens eine wäßrige Phase;
(ddd) Abtrennung der mindestens einen organischen Phase aus der in Stufe (ccc) erhaltenen mehrphasigen Mischung (M-ccc) unter Erhalt mindestens einer wäßrigen Phase (P-ddd), die in Stufe (aaa) rückgeführt wird;
(eee) Gegenstromextraktion der in Stufe (ddd) abgetrennten mindestens einen organischen Phase unter Verwendung der in Stufe (aaa) erhaltenen wäßrigen Phase (P-aaa) und unter Erhalt einer wäßrigen Phase (P-eee), die in Stufe (ccc) rückgeführt wird;
(fff) Neutralisation der mindestens einen, in Stufe (eee) erhaltenen organischen Phase unter Erhalt eines Gemisches (G-fff), umfassend mindestens ein Alkali- oder Erdalkalisalz, sowie mindestens den Alkohol (A), das Lösungsmittel (L) und den mindestens einen ungesättigten Alkohol (B);
(ggg) Abtrennung des mindestens einen Alkali- oder Erdalkalisalzes aus dem in Stufe (fff) erhaltenen Gemisch (G-fff) unter Erhalt eines Gemisches (G-ggg), umfassend mindestens den Alkohol (A), das Lösungsmittel (L) und den mindestens einen ungesättigten Alkohol (B);
(hhh) Destillation des in Stufe (ggg) erhaltenen Gemisches (G-ggg) unter Erhalt des mindestens einen ungesättigten Alkohols (B) in schmelzflüssiger Form. unter Erhalt einer Mischung (M-hhh), umfassend das Lösungsmittel (L) und den Alkohol (A), und unter Erhalt eines Gemisches (G-hhh), enthaltend geringe Mengen an dem mindestens einen ungesättigten Alkohol (B), wobei das Lösungsmittel (L) und der Alkohol (A) als Mischung in Stufe (aaa) rückgeführt werden und das Gemisch (G-hhh), enthaltend geringe Mengen an dem mindestens einen ungesättigten Alkohol (B), in Stufe (ggg) rückgeführt wird, und
(jjj) Herstellung eines pastillenförmigen Granulats aus der Schmelze, die den mindestens einen ungesättigten Alkohol (B) umfaßt und in (hhh) erhalten wird.

Darüber hinaus beschreibt die vorliegende Erfindung auch ein integriertes Verfahren zur Herstellung mindestens eines hydrierten Alkohols (C), das die kontinuierlich durchgeführten Stufen (aaa) bis (ggg), wie oben beschrieben, aufweist sowie die kontinuierlich durchgeführten Stufen (ggg'), (hhh') und (jjj') umfaßt, die nach der Stufe (ggg) durchgeführt werden:
(ggg')Hydrierung des mindestens einen ungesättigten Alkohols (B) in dem aus Stufe (ggg) erhaltenen Gemisch (G-ggg) unter Erhalt eines Gemischs (G-ggg'), umfassend mindestens den Alkohol (A), das Lösungsmittel (L) und mindestens einen hydrierten Alkohol (C);
(hhh')Destillation des in Stufe (ggg') erhaltenen Gemisches (G-ggg') unter Erhalt des mindestens einen hydrierten Alkohols (C) in schmelzflüssiger Form, unter Erhalt einer Mischung (M-hhh'), umfassend das Lösungsmittel (L) und den Alkohol (A), und unter Erhalt eines Gemisches (G-hhh'), enthaltend geringe Mengen an dem mindestens einen hydrierten Alkohol (C), wobei das Lösungsmittel (L) und der Alkohol (A) als Mischung in Stufe (aaa) rückgeführt werden und das Gemisch (G-hhh'), enthaltend geringe Mengen an dem mindestens einen hydrierten Alkohol (C), in Stufe (ggg) rückgeführt wird,
(jjj') Herstellung eines pastillenförmigen Granulats aus der Schmelze, die den mindestens einen gesättigten Alkohol (C) umfaßt und in (hhh') erhalten wird.

Selbstverständlich ist es auch denkbar, daß die Alkohole (B) oder (C), die in den Stufen (III) oder (III') bevorzugt in schmelzflüssiger Form anfallen, nicht direkt im Anschluß zu Tropfen ausgeformt werden. In diesem Fall kann die Schmelze beispielsweise gesammelt werden und zu einem späteren Zeitpunkt zu Tropfen ausgeformt werden. Dabei ist es wiederum denkbar, die Schmelze während der Lagerung erstarren zu lassen und vor der Ausformung zu Tropfen wieder zu verflüssigen. Ebenso ist es aber auch denkbar, die Schmelze während der Lagerung im flüssigen Zustand zu halten.

Weiter ist auch denkbar, daß der mindestens eine ungesättigte Alkohol (B) oder der mindestens eine gesättigte Alkohol (C) in Stufe (hhh) oder in Stufe (hhh') nicht in schmelzflüssiger Form anfallen. In diesem Fall werden (B) oder (C) zuerst in eine Schmelze überführt und anschließend die Schmelze zu Tropfen ausgeformt. Zur Herstellung der Schmelze von (B) oder (C) sind hierbei sämtliche geeignete Verfahren denkbar.

Wie obenstehend ausführlich beschrieben, stellt die vorliegende Erfindung ein Verfahren zur Herstellung eines pastillenförmigen Granulats zur Verfügung, in dem eine große Anzahl von Produkten, die aus den einzelnen Stufen und Schritten resultieren, wieder in das Verfahren rückgeführt werden. Damit wird ein kostensparendes und ökologisch effizientes Verfahren bereitgestellt, was durch die bevorzugte kontinuierliche Betriebsweise jeder Stufe und jedes Schrittes noch verbessert wird.

Als besonders bevorzugte Alkohole, die die Schmelze im erfindungsgemäßen Verfahren umfaßt, sind unter anderem Polyalkylenglykole wie beispielsweise Polyethylenglykole, Polypropylenglykole oder Trimethylolpropan zu nennen. Ebenso bevorzugt sind Cyclohexanole wie beispielsweise 1-Ethinyl-1-cyclohexanol, Hexandiole wie beispielsweise 1,6-Hexandiol, Butindiole wie beispielsweise 2-Butin-1,4-diol, Hexindiole wie beispielsweise 3-Hexin-2,5-diol und Neopentylglykol.

In einer weiter bevorzugten Ausführungsform betrifft die vorliegende Erfindung ein Verfahren, wie oben beschrieben, das dadurch gekennzeichnet ist, daß der Alkohol ausgewählt wird aus der Gruppe bestehend aus Dimethylhexindiol. Dimethylhexandiol, Neopentylglykol und Trimethylolpropan.

Hinsichtlich DMHD ist es insbesondere denkbar, daß diese Verbindung gemäß einem Verfahren, das die Schritte (I), (II), (II') und (III') umfaßt, hergestellt wird. Insbesondere ist weiter denkbar, daß Dimethylhexandiol durch Hydrierung von Dimethylhexindiol (DMHDY) hergestellt wird, das wiederum durch ein Verfahren bereitgestellt wird, das die Schritte (I), (II) und (III) umfaßt.

Selbstverständlich ist es auch möglich, DMHD nach allen anderen möglichen Verfahren herzustellen. So ist es beispielsweise denkbar, DMHD durch eine Radikalreaktion herzustellen. Bei dieser Reaktion werden beispielsweise Methylbutinol und Methylbutenol und Isopropanol unter Zusatz eines Radikalstarters wie beispielsweise Dibutylperoxid miteinander umgesetzt.

In einer bevorzugten Ausführungsform des erfindungsgemäßen Verfahrens wird die Schmelze, die mindestens einen Alkohol, wie oben beschrieben, umfaßt, derart zu Tropfen ausgeformt, daß keine Vorkristallisation stattfindet.

Mit dem Begriff "Vorkristallisation" werden im Rahmen der vorliegenden Erfindung ein oder mehrere Verfahrensschritte verstanden, in denen nach Herstellung der Schmelze der Kristallgehalt der Schmelze durch Zusatz von Kristallen zur Schmelze oder durch Verwendung einer Kristallisationsvorrichtung, in der Regel mit Wischern und Schabern ausgestattete Kühlflächen, wie beispielsweise in den beiden oben genannten Druckschriften beschrieben, oder durch Teilverdampfung der Schmelze oder einer Lösung erzeugt oder erhöht wird.

Demgemäß betrifft die vorliegende Erfindung auch ein Verfahren, wie oben beschrieben, das dadurch gekennzeichnet ist, daß die Schmelze vor der Ablage der Tropfen keiner Vorkristallisation unterworfen wird.

Die Schmelze, die zu Tropfen ausgeformt wird, kann im wesentlichen jeden beliebigen Kristallgehalt aufweisen, solange gewährleistet ist, daß die Schmelze zu Tropfen ausgeformt und auf der Kühlfläche abgelegt werden kann. In einer bevorzugten Ausführungsform des erfindungsgemäßen Verfahrens wird hierbei ein pastillenförmiges Granulat hergestellt, wobei die Schmelze einen Kristallgehalt von weniger als 3 Gew.-%, insbesondere von weniger als 2 Gew.-%, besonders bevorzugt von weniger als 1 Gew.-%, jeweils bezogen auf das Gesamtgewicht der Schmelze, und ganz besonders bevorzugt keine Kristalle aufweist.

Daher betrifft die vorliegende Erfindung auch ein Verfahren, wie oben beschrieben, das dadurch gekennzeichnet ist, daß die Schmelze einen Kristallgehalt von weniger als 3 Gew.-% aufweist.

Im allgemeinen ist das erfindungsgemäße Verfahren dazu geeignet, Schmelzen im wesentlichen jeder Viskosität zu Granulat zu verarbeiten, solange gewährleistet ist, daß die Schmelze zu Tropfen ausgeformt und auf der Kühlfläche abgelegt werden kann. Bevorzugt werden nach dem erfindungsgemäßen Verfahren Schmelzen zu Granulat verarbeitet, die eine Viskosität aufweisen, die im Bereich von 1 bis 1000 mPas, vorzugsweise im Bereich von 1 bis 100 mPas, bevorzugt im Bereich von 1 bis 20 mPas liegt.

Daher betrifft die vorliegende Erfindung auch ein Verfahren, wie oben beschrieben, das dadurch gekennzeichnet ist, daß die Schmelze eine Viskosität im Bereich von 1 bis 1000 mPas aufweist.

In einer weiter bevorzugten Ausführungsform des erfindungsgemäßen Verfahrens wird die Schmelze, die zu Tropfen ausgeformt wird, auf einer von der Schmelze schlecht benetzbaren Kühlfläche abgelegt.

Welches Material die Kühlfläche, die von der zu Tropfen ausgeformten Schmelze schlecht benetzbar ist, aufweist, ist im Rahmen der vorliegenden Erfindung im wesentlichen frei wählbar und kann nach der jeweiligen Zusammensetzung der Schmelze, die zu einem Granulat verarbeitet werden soll, ausgewählt werden. Selbstverständlich ist hierbei darauf zu achten, daß sich das Material der Kühlfläche gegenüber der jeweiligen Schmelze chemisch inert verhält und die notwendige Kühlung der Kühlfläche nicht verhindert. Unter diesen Voraussetzungen sind im wesentlichen alle geeigneten Materialien, die aus dem Stand der Technik bekannt sind, einsetzbar. In einer bevorzugten Ausführungsform ist das Material der Oberfläche der Kühlfläche, auf der die Tropfen abgelegt werden, aus Metall, weiter bevorzugt aus Stahl und ganz besonders bevorzugt aus Edelstahl gefertigt.

Daher betrifft die vorliegende Erfindung auch ein Verfahren, wie oben beschrieben, das dadurch gekennzeichnet ist, daß die Oberfläche der Kühlfläche, auf der die Tropfen gernäß (iii) abgelegt werden, aus Edelstahl gefertigt ist.

Durch die erfindungsgemäße spezifische Auswahl des Materials der Kühlfläche kann es in einfacher Weise erreicht werden, daß die zu Tropfen ausgeformte Schmelze auf der Kühlfläche nicht oder nur wenig spreitet, bevor die Schmelze zum Granulat erstarrt. Auf diese Art und Weise kann insbesondere auf Vorrichtungen und Verfahren verzichtet werden, die die Eigenschaften der Schmelze beeinflussen müssen, um die Form des Granulats steuern zu können. Insbesondere kann im Rahmen des erfindungsgemäßen Verfahrens, wie bereits beschrieben, auf eine Vorkristallisation der Schmelze verzichtet werden.

Was die Ausformung der Schmelze zu Tropfen anbelangt, so sind im Rahmen des erfindungsgemäßen Verfahrens sämtliche geeignete Verfahren und Vorrichtungen denkbar.

Insbesondere sind solche Vorrichtungen zu nennen, bei denen die Schmelze durch Druck die Schmelze taktweise über Ventile oder Schieber oder über taktweise freigegebene Öffnungen ausgetropft wird oder bei denen durch oszillierende oder rotierende Verdrängkörper die Schmelze ausgetropft oder ausgepreßt wird.

In einer bevorzugten Ausführungsform der vorliegenden Erfindung wird beispielsweise eine Schmelze in einem Behälter vorgelegt, der eine Auslaßöffnung aufweist, durch die die Schmelze austreten kann und zu einem Ventil geleitet wird, das so reguliert wird, daß die Schmelze tropfenweise auf eine darunterliegende Kühlfläche abgelegt wird. Hierbei kann die Tropfenerzeugung beispielsweise dadurch gesteuert werden, daß das Ventil taktweise geöffnet wird. Sowohl der Behälter an sich als auch die Leitungen, falls vorhanden, als auch das Ventil selbst können beheizt sein, um die Schmelze entsprechend temperieren zu können. Hierbei ist es unter anderem denkbar, daß die Schmelze überall dieselbe Temperatur aufweist. Ebenso ist es aber auch möglich, daß Behälter, Leitungen, falls vorhanden, und Ventil selbst unterschiedlich temperiert sind.

Weitere Vorrichtungen zur Ausformung von Tropfen aus einer Schmelze sind beispielsweise in der DE-C 34 21 625, der DE-C 29 41 802 und der DE-B 28 53 054 beschrieben, die diesbezüglich durch Bezugnahme in den Inhalt der vorliegenden Anmeldung aufgenommen werden.

Im Rahmen des erfindungsgemäßen Verfahrens werden solche Vorrichtungen zur Tropfenerzeugung bevorzugt eingesetzt, die mehrere Abtropfstellen zur gleichzeitigen Erzeugung mehrerer Tropfen aufweisen.

Bei der Herstellung des Granulates aus der Schmelze nach dem erfindungsgemäßen Verfahren können im wesentlichen alle geeigneten Temperaturen, die die Schmelze aufweist, gewählt werden. Gleiches gilt für die Temperaturen, die die Kühlfläche aufweist.

Besonders bevorzugt werden Aufgabetemperaturen der Schmelze, die im allgemeinen im Bereich bis zu 100 °C, bevorzugt im Bereich bis zu 30 °C und besonders bevorzugt im Bereich bis zu 15 °C über der Schmelztemperatur der Schmelze liegen.

Daher betrifft die vorliegende Erfindung auch ein Verfahren, wie oben beschrieben, das dadurch gekennzeichnet ist, daß die Temperatur, die die zu Tropfen ausgeformte Schmelze bei Aufgabe auf die Kühlfläche aufweist, bis zu 100 °C über der Schmelztemperatur der Schmelze liegt.

Die Temperatur der Kühlfläche, auf der die zu Tropfen ausgeformte Schmelze abgelegt wird, wird im allgemeinen so gewählt, daß die Schmelze mit der gewünschten Geschwindigkeit abkühlt.

Im allgemeinen liegen diese Temperaturen der Kühlfläche im Bereich von -30 bis +80 °C, bevorzugt im Bereich von 0 bis +50 °C und besonders bevorzugt im Bereich von +15 bis +40 °C.

Daher betrifft die vorliegende Erfindung auch ein Verfahren, wie oben beschrieben, das dadurch gekennzeichnet ist, daß die Kühlfläche, auf der die zu Tropfen ausgeformte Schmelze abgelegt wird, eine Temperatur im Bereich von -30 bis +80 °C aufweist.

Gemäß des erfindungsgemäßen Verfahrens kann in Abhängigkeit von unter anderem der Temperatur der Schmelze beim Ablegen des Tropfens, der Tropfenform, dem Tropfenvolumen und der Temperatur der Kühlfläche ein pastillenförmiges Granulat mit unterschiedlichen Geometrien hergestellt werden. Durch Variation unter anderem der vorgenannten Parameter sind beispielsweise Höhe und Durchmesser der Pastillen den Erfordernissen der weiteren Verwendung anpaßbar.

Im allgemeinen liegt der mittlere Durchmesser des nach dem erfindungsgemäßen Verfahren hergestellten Granulats im Bereich von 1 bis 20 mm, bevorzugt im Bereich von 3 bis 10 mm und besonders bevorzugt im Bereich von 4 bis 8 mm. Die mittlere Höhe des nach dem erfindungsgemäßen Verfahren hergestellten Granulats liegt im allgemeinen im Bereich von 0,5 bis 5 mm, bevorzugt im Bereich von 1 bis 4 mm und besonders bevorzugt im Bereich von 1,5 bis 3 mm.

Daher betrifft die vorliegende Erfindung auch ein Granulat, wie oben beschrieben, das dadurch gekennzeichnet ist, daß es einen mittleren Durchmesser im Bereich von 1 bis 20 mm und eine mittlere Höhe im Bereich von 0,5 bis 5 mm aufweist.

In einer ganz besonders bevorzugten Ausführungsform werden pastillenförmige Granulate hergestellt, die im wesentlichen aus DMHD oder DMHDY oder Neopentylglykol oder Trimethylolpropan bestehen. Im allgemeinen liegt in diesem Fall der Gehalt des pastillenförmigen Granulats an DMHD oder DMHDY oder Neopentylglykol oder Trimethylolpropan bei mehr als 90 Gew.-%, bevorzugt bei mehr als 95 Gew.-% und besonders bevorzugt bei mehr als 99 Gew.-%, jeweils bezogen auf das Gesamtgewicht des Granulats.

Daher betrifft die vorliegende Erfindung auch ein Granulat, wie oben beschrieben, das dadurch gekennzeichnet ist, daß es zu mehr als 90 Gew.-%, bezogen auf das Gesamtgewicht des Granulats, aus Dimethylhexindiol oder Dimethylhexandiol oder Neopentylglykol oder Trimethylolpropan besteht.

Ein besonderer Vorteil des erfindungsgemäßen Granulats ist darin zu sehen, daß es, verglichen mit beispielsweise schuppenförmigem Granulat, eine geringe Verfestigungs- bzw. Verharschungsneigung besitzt. Produkte mit hoher Verfestigungs- bzw. Verharschungsneigung sind jedoch unter anderem schwer handhabbar und schlecht lagerfähig.

Daher betrifft die vorliegende Erfindung auch ein Granulat, wie oben beschrieben, das dadurch gekennzeichnet ist, daß es eine geringe Verfestigungsneigung aufweist.

Im Vergleich zu beispielsweise schuppenförmigem Granulat weist das erfindungsgemäße pastillenförmige Granulat auch einen verminderten Staubanteil auf. Unter "Staub" werden im Rahmen der vorliegenden Erfindung Partikel verstanden, die die gleiche Zusammensetzung wie das Granulat aufweisen, jedoch eine Größe aufweisen, die unter der des Granulats liegt und für die Handhabung und Anwendung des Granulats nicht erwünscht ist.

Im folgenden wird die Erfindung durch ein Ausführungsbeispiel illustriert.

### Beispiel:

Eine DMHD-Schmelze mit einer Reinheit von 99,85 %, bestimmt als Flächenprozente aus gaschromatographischer Analyse, wurde unter Überlagerung von Stickstoff in einem 11-Behälter drucklos bei einer Temperatur von 102 °C vorgelegt. Der Behälter war zur Temperierung der Schmelze mit einem Doppelmantel ausgestattet und wurde entsprechend temperiert. Am Boden des Behälters befand sich ein Auslaufrohr, versehen mit einem Feinregulierventil, die beide ebenfalls beheizt waren.

Durch taktweises Öffnen des Ventils erfolgte ein Austropfen der Schmelze. Dabei befand sich die Austropfstelle 10 mm über der für die Erstarrung des Tropfens verwendeten Kühlfläche. Die Kühlfläche bestand aus Edelstahl und wurde mit Kühlwasser auf eine Temperatur von 25 °C eingestellt. Die Tropfen wurden nacheinander auf die Kühlfläche abgelegt. Durch Erstarren entstanden Pastillen, die einen Durchmesser von 5 bis 7 mm und eine Dicke von 1,8 bis 2,7 mm aufwiesen.

Die gleiche DMHD-Schmelze wurde auf eine Kühlwalze zur Erzeugung von Schuppen aufgegeben. Die Kühlwalze hatte einen Durchmesser von 200 mm und eine Breite von 100 mm.

Die Walze wurde in eine Schmelzwanne eingetaucht, in der die DMHD-Schmelze bei einer Temperatur von 110 °C vorgelegt war. Die Kühlwalze wurde mit Kühlwasser bei einer Temperatur von 25 °C gekühlt. Bei einer Walzendrehzahl von 2/min wurde die Schmelze auf die Walze aufgezogen, erstarrt und nach Erstarrung durch eine Vorrichtung abgeschabt. Die dabei entstandenen Schuppen wiesen eine Dicke von 0,6 bis 0,7 mm und eine unregelmäßige Größe von wenigen Quadratmillimetern bis etwa Fingemagelgröße auf. Die Schuppenware enthielt im Gegensatz zur pastillenförmigen Granulatware einen signifikanten Staubanteil mit Partikeln < 1 mm.

Die erhaltenen Schuppen und Pastillen wurden in einem Vergleichstest auf das Verbackungsverhalten hin untersucht. Hierzu wurden jeweils 30 g Schuppen und Pastillen in zylindrische Metallgefäße (Innendurchmesser 43 mm, Höhe 50 mm) gefüllt.

Nach Egalisieren des Schüttkonus wurden die in den Metallgefäßen befindlichen Proben mit Metallstempeln (Gewicht: je 455 g) von oben belastet. Die belasteten Proben wurden bei Raumtemperatur (Umgebungsluft im Labor) über 14 Tage ruhend gelagert. Nach Ablauf der Lagerzeit wurden die Stempel abgenommen und der Grad der Verfestigung der Schuppen bzw. der Pastillen durch Umdrehen der zylindrischen Gefäße und Erfassung des von selbst ausfließenden Anteils der Schuppen bzw. der Pastillen geprüft. Dabei wurden die in der folgenden Tabelle 1 angegebenen Ergebnisse erzielt:

**Tabelle 1**

| **Proben-Nummer** | **Ausflußanteil / Gew.-%** |
|---|---|
| Pastillen Nr. 1 | 24 |
| Pastillen Nr. 2 | 17 |
| Pastillen Nr. 3 | 32 |
| Schuppen Nr. 1 | 1,9 |
| Schuppen Nr. 2 | 0,7 |
| Schuppen Nr. 3 | 1,6 |

Darüber hinaus war festzustellen, daß der nicht frei ausfließende Teil der Pastillen bereits bei leichtem Anstoßen des zylindrischen Gefäßes als dispergiertes Granulat ausfloß, während die Schuppen erst nach festerem Anstoßen und dann als weitgehend zusammenhängendes Agglomerat herausfielen.

## Patentansprüche

1. Verfahren zur Herstellung eines pastillenförmigen Granulats aus einer Schmelze, wobei
(i) eine Schmelze bereitgestellt wird,
(ii) die bereitgestellte Schmelze zu Tropfen ausgeformt wird,
(iii) die Tropfen auf einer Kühlfläche abgelegt werden und
(iv) die abgelegten Tropfen zu dem Granulat erstarren,
**dadurch gekennzeichnet, daß** die Schmelze einen Alkohol umfaßt und die Schmelze eine Schmelztemperatur von 30 °C und darüber aufweist.

2. Verfahren nach Anspruch 1, **dadurch gekennzeichnet, daß** der Alkohol ausgewählt wird aus der Gruppe bestehend aus
(a) einem Alkohol (B), der durch ein Verfahren bereitgestellt wird, das die folgenden Stufen (I) bis (III) umfaßt:
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(III) Destillation des in Stufe (II) erhaltenen Gemisches (G-II) unter Erhalt des mindestens einen Alkohols (B) sowie eines Gemisches (G-III), umfassend das Lösungsmittel (L) und den Alkohol (A),
wobei das in Stufe (III) erhaltene Lösungmittel (L) und der in Stufe (III) erhaltene Alkohol (A) als Mischung in Stufe (I) rückgeführt werden,
(b) einem Alkohol (C), der durch ein Verfahren bereitgestellt wird, das die folgenden Stufen (I) bis (III') umfaßt:
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(II') Hydrierung mindestens eines ungesättigten Alkohols (B) in dem aus Stufe (II) erhaltenen Gemisch (G-II) unter Erhalt eines Gemisches (G-II'), umfassend mindestens einen hydrierten Alkohol (C), den Alkohol (A) und das Lösungsmittel (L);
(III') Destillation des in Stufe (II') erhaltenen Gemisches (G-II') unter Erhalt des mindestens einen Alkohols (C) sowie eines Gemisches (G-III'), umfassend das Lösungsmittel (L) und den Alkohol (A),
wobei das in Stufe (III') erhaltene Lösungsmittel (L) und der in Stufe (III') erhaltene Alkohol (A) als Mischung in Stufe (I) rückgeführt werden, und
(c) einem Gemisch aus zwei oder mehr davon.

3. Verfahren nach Anspruch 1 oder 2, **dadurch gekennzeichnet, daß** der Alkohol ausgewählt wird aus der Gruppe bestehend aus Dimethylhexindiol, Dimethylhexandiol, Neopentylglykol und Trimethylolpropan.

4. Verfahren nach einem der Ansprüche 1 bis 3, **dadurch gekennzeichnet, daß** die Schmelze mindestens eine Eigenschaft ausgewählt aus der folgenden Gruppe, umfassend:
(i) die Schmelze wird vor der Ablage der Tropfen keiner Vorkristallisation unterworfen,
(ii) die Schmelze weist einen Kristallgehalt von weniger als 3 Gew.-% auf,
(iii) die Schmelze weist eine Viskosität im Bereich von 1 bis 1000 mPas auf.

5. Verfahren nach einem der Ansprüche 1 bis 4, **dadurch gekennzeichnet, daß** die Oberfläche der Kühlfläche, auf der die Tropfen gemäß (iii) abgelegt werden, aus Edelstahl gefertigt ist.

6. Verfahren nach einem der Ansprüche 1 bis 5, **dadurch gekennzeichnet, daß** die Temperatur, die die zu Tropfen ausgeformte Schmelze bei Aufgabe auf die Kühlfläche aufweist, bis zu 100 °C über der Schmelztemperatur der Schmelze liegt.

7. Verfahren nach einem der Ansprüche 1 bis 6, **dadurch gekennzeichnet, daß** die Kühlfläche, auf der die zu Tropfen ausgeformte Schmelze abgelegt wird, eine Temperatur im Bereich von -30 bis +80 °C aufweist.

8. Pastillenförmiges Granulat, herstellbar durch ein Verfahren, in dem
(i) eine Schmelze bereitgestellt wird,
(ii) die bereitgestellte Schmelze zu Tropfen ausgeformt wird,
(iii) die Tropfen auf einer Kühlfläche abgelegt werden und
(iv) die abgelegten Tropfen zu dem Granulat erstarren,
**dadurch gekennzeichnet, dass** die Schmelze eine Schmelztemperatur von 30 °C und darüber aufweist, sowie die Schmelze einen Alkohol umfaßt, welcher ausgewählt wird aus der Gruppe bestehend aus
(a) einem Alkohol (B), der durch ein Verfahren bereitgestellt wird, das die folgenden Stufen (I) bis (III) umfaßt:
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(III) Destillation des in Stufe (II) erhaltenen Gemisches (G-II) unter Erhalt des mindestens einen Alkohols (B) sowie eines Gemisches (G-III), umfassend das Lösungsmittel (L) und den Alkohol (A),
wobei das in Stufe (III) erhaltene Lösungmittel (L) und der in Stufe (III) erhaltene Alkohol (A) als Mischung in Stufe (I) rückgeführt werden,
(b) einem Alkohol (C), der durch ein Verfahren bereitgestellt wird, das die folgenden Stufen (I) bis (III') umfaßt :
(I) Umsetzung mindestens eines Alkali- oder Erdalkalihydroxids mit mindestens einem Alkohol (A) in mindestens einem organischen Lösungsmittel (L) unter Erhalt eines Gemisches (G-I), umfassend mindestens den Alkohol (A), das Lösungsmittel (L) und ein Alkoholat (AL);
(II) Umsetzung mindestens einer Carbonylverbindung der allgemeinen Struktur R-CO-R' mit mindestens einem Alkin der allgemeinen Struktur R"-C≡C-H und dem in Stufe (I) erhaltenen Gemisch (G-I) unter Erhalt eines Gemisches (G-II), umfassend mindestens den Alkohol (A), das Lösungsmittel (L) und einen ungesättigten Alkohol (B);
(II') Hydrierung mindestens eines ungesättigten Alkohols (B) in dem aus Stufe (II) erhaltenen Gemisch (G-II) unter Erhalt eines Gemisches (G-II'), umfassend mindestens einen hydrierten Alkohol (C), den Alkohol (A) und das Lösungsmittel (L);
(III') Destillation des in Stufe (II') erhaltenen Gemisches (G-II') unter Erhalt des mindestens einen Alkohols (C) sowie eines Gemisches (G-III'), umfassend das Lösungsmittel (L) und den Alkohol (A),
wobei das in Stufe (III') erhaltene Lösungsmittel (L) und der in Stufe (III') erhaltene Alkohol (A) als Mischung in Stufe (I) rückgeführt werden, und
(c) einem Gemisch aus zwei oder mehr davon.

9. Granulat nach Anspruch 8, **dadurch gekennzeichnet, daß** es einen mittleren Durchmesser im Bereich von 1 bis 20 mm und eine mittlere Höhe im Bereich von 0,5 bis 5 mm aufweist.

10. Granulat nach Anspruch 9 oder 10, **dadurch gekennzeichnet, daß** es zu mehr als 90 Gew.-%, bezogen auf das Gesamtgewicht des Granulats, aus Dimethylhexindiol oder Dimethylhexandiol oder Neopentylglykol oder Trimethylolpropan besteht.

## Claims

1. A process for the production of tablet-shaped granular material from a melt, in which
(i) a melt is prepared,
(ii) the prepared melt is shaped to droplets,
(iii) the droplets are deposited on a cooling surface, and
(iv) the deposited droplets solidify to give the granular material,
wherein the melt comprises an alcohol, and the melt has a melting point of 30°C or above.

2. A process as claimed in claim 1, wherein the alcohol is selected from the group consisting of
(a) an alcohol (B) which is prepared by a process which comprises the following stages (I) to (III):
(I) reaction of at least one alkali or alkaline earth metal hydroxide with at least one alcohol (A) in at least one organic solvent (L) to give a mixture (G-I) comprising at least the alcohol (A), the solvent (L) and an alkoxide (AL) ;
(II) reaction of at least one carbonyl compound of the general structure R-CO-R' with at least one alkyne of the general structure R"-C≡(C-H and the mixture (G-I) obtained in stage (I) to give a mixture (G-II) comprising at least the alcohol (A), the solvent (L) and an unsaturated alcohol (B);
(III) distillation of the mixture (G-II) obtained in stage (II) to give the at least one alcohol (B) and a mixture (G III) comprising the solvent (L) and the alcohol (A),
where the solvent (L) obtained in stage (III) and the alcohol (A) obtained in stage (III) are recycled into stage (I) as a mixture,
(b) an alcohol (C) which is prepared by a process which comprises the following stages (I) to (III'):
(I) reaction of at least one alkali or alkaline earth metal hydroxide with at least one alcohol (A) in at least one organic solvent (L) to give a mixture (G-I) comprising at least the alcohol (A), the solvent (L) and an alkoxide (AL);
(II) reaction of at least one carbonyl compound of the general structure R-CO-R' with at least one alkyne of the general structure R"-C(C-H and the mixture (G-I) obtained in stage (I) to give a mixture (G-II) comprising at least the alcohol (A), the solvent (L) and an unsaturated alcohol (B);
(II') hydrogenation of at least one unrated alcohol (B) in the mixture (G-II) obtained from stage (II) to give a mixture (G-II') comprising at least one hydrogenated alcohol (C), the alcohol (A) and the solvent (L);
(III') distillation of the mixture (G-II') obtained in stage (II') to give the at least one alcohol (C) and a mixture (GIII') comprising the solvent (L) and the alcohol (A),
where the solvent (L) obtained in stage (III') and the alcohol (A) obtained in stage (III') are recycled into stage (I) as a mixture, and
(c) a mixture of two or more thereof.

3. A process as claimed in claim 1 or 2, wherein the alcohol is selected from the group consisting of dimethylhexynediol, dimethylhexanediol, neopentyl glycol and trimethylolpropane.

4. A process as claimed in one of claims 1 to 3, wherein the melt has at least one property selected from the following group:
(i) the melt is not subjected to precrystallization before deposition of the droplets,
(ii) the melt has a crystal content of less than 3% by weight,
(iii) the melt has a viscosity in the range from 1 to 1000 mPas.

5. A process as claimed in one of claims 1 to 4, wherein the cooling surface on which the droplets are deposited in (iii) is made of stainless steel.

6. A process as claimed in one of claims 1 to 5, wherein the temperature of the melt shaped to give droplets on application to the cooling surface is up to 100°C above the melting point of the melt.

7. A process as claimed in one of claims 1 to 6, wherein the cooling surface on which the melt shaped to give droplets is deposited has a temperature in the range from -30 to +80°C.

8. A tablet-shaped granular material which can be produced by a process in which
(i) a melt is prepared,
(ii) the prepared melt is shaped to droplets,
(iii) the droplets are deposited on a cooling surface, and
(iv) the deposited droplets solidify to give the granular material,
wherein the melt has a melting point of 30°C or above, and the melt comprises an alcohol which is selected from the group consisting of
(a) an alcohol (B) which is prepared by a process which comprises the following stages (I) to (III):
(I) reaction of at least one alkali or alkaline earth metal hydroxide with at least one alcohol (A) in at least one organic solvent (L) to give a mixture (G-I) comprising at least the alcohol (A), the solvent (L) and an alkoxide (AL);
(II) reaction of at least one carbonyl compound of the general structure R-CO-R' with at least one alkyne of the general structure R"-C≡(C-H and the mixture (G-I) obtained in stage (I) to give a mixture (G-II) comprising at least the alcohol (A), the solvent (L) and an unsaturated alcohol (B);
(III) distillation of the mixture (G-II) obtained in stage (II) to give the at least one alcohol (B) and a mixture (G III) comprising the solvent (L) and the alcohol (A),
where the solvent (L) obtained in stage (III) and the alcohol (A) obtained in stage (III) are recycled into stage (I) as a mixture,
(b) an alcohol (C) which is prepared by a process which comprises the following stages (I) to (III'):
(I) reaction of at least one alkali or alkaline earth metal hydroxide with at least one alcohol (A) in at least one organic solvent (L) to give a mixture (G-I) comprising at least the alcohol (A), the solvent (L) and an alkoxide (AL);
(II) reaction of at least one carbonyl ompound of the general structure R R' with at least one alkyne of the general structure R"-C(C-H and the mixture (G-I) obtained in stage (I) to give a mixture (G-II) comprising at least the alcohol (A), the solvent (L) and an unsaturated alcohol (B);
(II') hydrogenation of at least one unrated alcohol (B) in the mixture (G-II) obtained from stage (II) to give a mixture (G-II') comprising at least one hydrogenated alcohol (C), the alcohol (A) and the solvent (L);
(III') distillation of the mixture (G-II') obtained in stage (II') to give the at least one alcohol (C) and a mixture (G III') comprising the solvent (L) and the alcohol (A),
where the solvent (L) obtained in stage (III') and the alcohol (A) obtained in stage (III') are recycled into stage (I) as a mixture, and
(c) a mixture of two or more thereof.

9. A granular material as claimed in claim 8, which has a mean diameter in the range from 1 to 20 mm and a mean height in the range from 0.5 to 5 mm.

10. A granular material as claimed in claim 8 or 9, which comprises more than 90% by weight, based on the total weight of the granular material, of dimethyl hexynediol or dimethylhexanediol or neopentyl glycol or trimethyolpropane.

## Revendications

1. Procédé de fabrication d'un granulé en forme de pastille à partir d'une masse fondue, dans lequel
(i) une masse fondue est mise à disposition,
(ii) la masse fondue mise à disposition est transformée en gouttes,
(iii) les gouttes sont déposées sur une surface de refroidissement, et
(iv) les gouttes déposées se solidifient pour donner le granulé,
**caractérisé en ce que** la masse fondue comprend un alcool, et que la masse fondue a une température de fusion de 30°C et plus.

2. Procédé selon la revendication 1, **caractérisé en ce que** l'alcool est choisi dans l'ensemble comprenant
(a) un alcool (B), qui est préparé par un procédé qui comprend les étapes (I) à (III) ci-après :
(I) réaction d'au moins un hydroxyde d'un métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) réaction d'au moins un composé carbonylé de structure générale R-CO-R' avec au moins un alcyne de structure générale R"-C≡C-H et le mélange (G-I) obtenu dans l'étape (I), avec obtention d'un mélange (G-II) comprenant au moins l'alcool (A), le solvant (L) et un alcool insaturé (B) ;
(III) distillation du mélange (G-II) obtenu dans l'étape (II), avec obtention d'au moins un alcool (B), ainsi que d'un mélange (G-III), comprenant le solvant (L) et l'alcool (A),
le solvant (L) obtenu dans l'étape (III) et l'alcool (A) obtenu dans l'étape (III) étant renvoyé sous forme d'un mélange à l'étape (I),
(b) un alcool (C), qui est préparé par un procédé qui comprend les étapes (I) à (III') ci-après :
(I) réaction d'au moins un hydroxyde d'un métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) réaction d'au moins un composé carbonylé de structure générale R-CO-R' avec au moins un alcyne de structure générale R"-C≡C-H et le mélange (G-I) obtenu dans l'étape (I), avec obtention d'un mélange (G-II) comprenant au moins l'alcool (A), le solvant (L) et un alcool insaturé (B) ;
(II') hydrogénation d'au moins un alcool insaturé (B) dans le mélange (G-II) obtenu dans l'étape (II), avec obtention d'un mélange (G-II') comprenant au moins un alcool hydrogéné (C), l'alcool (A) et le solvant (L) ;
(III') distillation du mélange (G-II') obtenu dans l'étape (II'), avec obtention d'au moins un alcool (C) ainsi que d'un mélange (G-III') comprenant le solvant (L) et l'alcool (A),
le solvant (L) obtenu dans l'étape (III') et l'alcool (A) obtenu dans l'étape (III') étant renvoyé sous forme d'un mélange à l'étape (I), et
(c) un mélange d'au moins deux d'entre eux.

3. Procédé selon la revendication 1 ou 2, **caractérisé en ce que** l'alcool est choisi dans l'ensemble comprenant le diméthylhexynediol, le diméthylhexanediol, le néopentylglycol et le triméthylolpropane.

4. Procédé selon l'une des revendications 1 à 3, **caractérisé en ce que** la masse fondue présente au moins une propriété choisie dans l'ensemble suivant :
(i) la masse fondue, avant dépose des gouttes, n'est soumise à aucune précristallisation,
(ii) la masse fondue présente une teneur en cristaux inférieure à 3 % en poids,
(iii) la masse fondue présente une viscosité comprise dans la plage de 1 à 1000 mPa.s.

5. Procédé selon l'une des revendications 1 à 4, **caractérisé en ce que** la face supérieure de la surface de refroidissement sur laquelle sont déposées les gouttes selon (iii) est fabriquée en acier inoxydable.

6. Procédé selon l'une des revendications 1 à 5, **caractérisé en ce que** la température que présente la masse fondue, transformée en gouttes, lors de sa mise en place sur la surface de refroidissement, va jusqu'à 100°C au-delà de la température de fusion de la masse fondue.

7. Procédé selon l'une des revendications 1 à 6, **caractérisé en ce que** la surface de refroidissement sur laquelle est déposée la masse fondue transformée en gouttes présente une température comprise dans la plage de -30 à +80°C.

8. Granulés en forme de pastilles, pouvant être fabriqués par un procédé dans lequel
(i) une masse fondue est mise à disposition,
(ii) la masse fondue mise à disposition est transformée en gouttes,
(iii) les gouttes sont déposées sur une surface de refroidissement, et
(iv) les gouttes déposées se solidifient pour donner le granulé,
**caractérisé en ce que** la masse fondue présente une température de fusion de 30°C et plus, et de plus la masse fondue comprend un alcool, qui est choisi dans l'ensemble comprenant :
(a) un alcool (B), qui est préparé par un procédé qui comprend les étapes (I) à (III) ci-après :
(I) réaction d'au moins un hydroxyde d'un métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) réaction d'au moins un composé carbonylé de structure générale R-CO-R' avec au moins un alcyne de structure générale R"-C≡C-H et le mélange (G-I) obtenu dans l'étape (I), avec obtention d'un mélange (G-II) comprenant au moins l'alcool (A), le solvant (L) et un alcool insaturé (B) ;
(III) distillation du mélange (G-II) obtenu dans l'étape (II), avec obtention d'au moins un alcool (B), ainsi que d'un mélange (G-III), comprenant le solvant (L) et l'alcool (A),
le solvant (L) obtenu dans l'étape (III) et l'alcool (A) obtenu dans l'étape (III) étant renvoyé sous forme d'un mélange à l'étape (I),
(b) un alcool (C), qui est préparé par un procédé qui comprend les étapes (I) à (III') ci-après :
(I) réaction d'au moins un hydroxyde d'un métal alcalin ou alcalino-terreux avec au moins un alcool (A) dans au moins un solvant organique (L), avec obtention d'un mélange (G-I) comprenant au moins l'alcool (A), le solvant (L) et un alcoolate (AL) ;
(II) réaction d'au moins un composé carbonylé de structure générale R-CO-R' avec au moins un alcyne de structure générale R"-C≡C-H et le mélange (G-I) obtenu dans l'étape (I), avec obtention d'un mélange (G-II) comprenant au moins l'alcool (A), le solvant (L) et un alcool insaturé (B) ;
(II') hydrogénation d'au moins un alcool insaturé (B) dans le mélange (G-II) obtenu dans l'étape (II), avec obtention d'un mélange (G-II') comprenant au moins un alcool hydrogéné (C), l'alcool (A) et le solvant (L) ;
(III') distillation du mélange (G-II') obtenu dans l'étape (II'), avec obtention d'au moins un alcool (C) ainsi que d'un mélange (G-III') comprenant le solvant (L) et l'alcool (A),
le solvant (L) obtenu dans l'étape (III') et l'alcool (A) obtenu dans l'étape (III') étant renvoyé sous forme d'un mélange à l'étape (I), et
(c) un mélange d'au moins deux d'entre eux.

9. Granulé selon la revendication 8, **caractérisé en ce qu'**il a un diamètre moyen compris dans la plage de 1 à 20 mm et une hauteur moyenne comprise dans la plage de 0,5 à 5 mm.

10. Granulé selon la revendication 9 ou 10, **caractérisé en ce qu'**il est constitué de plus de 90 % en poids, par rapport au poids total du granulé, de diméthylhexynediol ou de diméthylhexanediol ou de néopentylglycol ou de triméthylolpropane.
